# DEMANDE DE BREVET EUROPEEN

(11) **EP 2 295 026 A1**
(43) Date de publication de la demande: **16.03.2011**
(21) Numéro de dépôt: 10174334.2
(22) Date de dépôt: 27.08.2010
(51) Int. Cl.: A61K 8/31, A61K 8/97, A61Q 19/00

(54) **Procédé d'obtention d'un extrait végétal, extrait végétal obtenu selon le procédé et composition cosmétique ou dermatologique contenant l'extrait**

(30) Priorité: 01.09.2009 FR 0955956
(71) Demandeur: L'Oréal, 75008 Paris (FR)
(72) Inventeur: Grandjon, Vincent, 95200, Sarcelles (FR)
(74) Mandataire: Boulard, Denis

(57) **Abrégé**

L'invention concerne un procédé d'obtention d'un extrait végétal consistant à : i) mettre en contact au moins un matériau végétal avec un solvant ou un mélange de solvants comprenant au moins un alcane linéaire volatil d'origine végétale ; et ii) éliminer les résidus de matériau végétal, notamment par filtration.

## Description

La présente invention concerne le domaine des extraits végétaux entrant dans la formulation notamment de compositions pharmaceutiques, cosmétiques et/ou parfumerie ou alimentaires (actifs ou odorants). L'invention concerne en particulier un procédé d'obtention de tels extraits végétaux.

Les plantes et les produits naturels sont la source de molécules qui peuvent êtres utilisées sous forme d'extraits dans de nombreux domaines : pharmaceutique, cosmétique, parfumerie, aromatique ou alimentaire. Il peut s'agir extraits parfumants (principes odorants d'origine végétale : absolu de jasmin, d'iris, lactones, extrait de fleurs d'oranger... etc.), présentant des propriétés odorantes particulières recherchées.

Il peut s'agir d'extraits cosmétique (principes actifs d'origine végétale : huile d'immortelle, huile de germes de blé... etc.), présentant une activité cosmétique particulière (régénération cellulaire, activité anti-âge), de substances utilisées comme additifs (colorants : rouge de cochenille, bêta carotène, curcumine ; antioxydants : extraits de romarin, huiles végétales riches en acides gras poly-insaturés, conservateurs : extrait de pépins de pamplemousse) ou pour d'autres applications comme les insecticides naturels (pyréthrines, roténone).

Le séchage a certainement été le premier moyen utilisé pour conserver l'activité des plantes mais cette technique ne permet ni de concentrer ni de préserver les composés actifs de l'oxydation.

Au fil du temps, de nombreuses méthodes comme l'extraction à l'eau, la macération, l'hydrodistillation, la lixiviation, la décoction, l'enfleurage, l'extraction par solvants ont été utilisées pour obtenir des extraits de plantes.

L'hydrodistillation ou entraînement à la vapeur est une technique classique limitée à l'extraction des huiles essentielles. Ce procédé utilise une grande quantité d'énergie pour produire la vapeur qui entraîne les composés volatils et l'eau nécessaire pour condenser les vapeurs est rejetée sous forme d'effluent chaud entraînant également des pertes en énergie. Les huiles essentielles ainsi obtenues sont soumises à des températures voisines de 100°C. Les composants sensibles à la chaleur ou à l'hydrolyse sont souvent altérés.

L'extraction mettant en oeuvre des solvants organiques de synthèse ou issus des processus de raffinage de la pétrochimie, est la méthode qui présente la plus grande importance du point de vue industriel en raison de la standardisation des méthodes, de l'exigence de reproductibilité et de caractérisation des extraits obtenus.

Parmi ces procédés, ceux mettant en oeuvre de l'eau, de l'alcool ou des mélanges hydroalcooliques permettent d'obtenir des molécules essentiellement hydrophiles (ou fortement polaires) mais ces solvants entraînent également de nombreuses molécules de faible intérêt, qui peuvent diminuer l'activité des extraits (protéines provoquant des précipitations, tanins, minéraux), ou nuire à leur stabilité (anthocyanes).

D'autres solvants comme le glycérol et les dérivés du glycol, utilisés notamment en cosmétique, ont un très faible pouvoir solvant, ainsi les extraits obtenus sont pauvres en molécules actives.

Parmi les solvants organiques classiquement mis en oeuvre, on citera également les éthers de glycol, l'hexane, l'éther, l'acétone, le chlorure de méthylène, les alcools, les hydrocarbures aliphatiques ou aromatiques, les solvants chlorés et fluorés ou des molécules de synthèse comme la N-méthylpyrrolidone ou le dimethoxymethane, par exemple.

Ces solvants, s'ils permettent d'obtenir des molécules lipophiles (ou fortement apolaires) avec des rendements satisfaisants présentent de nombreux inconvénients. Ils sont notamment très inflammables, dangereux ou extrêmement toxiques pour l'homme et son environnement.

Les solvants synthétiques comme le perchloroéthylène, le chlorure de méthylène, les solvants chlorés et certaines fractions issues du pétrole comme l'hexane ont été très largement utilisés pour l'extraction d'arômes, de parfums, de colorants et de principes actifs de plantes. Souvent très toxiques, contenant des impuretés ou des dénaturants cancérigènes, ces solvants organiques sont particulièrement visés par les règlements internationaux qui imposent des teneurs en solvant résiduel très basses (de l'ordre de 1 à 5 ppm).

Ces règlements qui limitent ou interdisent leur utilisation pour des applications alimentaires ou thérapeutiques impliquent des retraitements coûteux pour éliminer les solvants résiduels, comme un chauffage supplémentaire, et les principes actifs peuvent être dégradés. De plus, lorsqu'ils sont mal choisis ou choisis uniquement sur des critères économiques, la nature chimique de ces solvants peut rendre les produits actifs instables.

C'est donc un objet de l'invention que de fournir un nouveau procédé d'obtention d'un extrait végétal mettant en oeuvre un solvant d'extraction ne présentant pas les problèmes discutés ci-avant en référence aux procédés connus.

C'est en particulier un objet de l'invention que de fournir un procédé d'extraction permettant l'obtention d'extraits végétaux à des coûts compatibles avec les exigences du marché des principes actifs, et permettant aux extraits de satisfaire aux exigences des produits d'origine naturel ou de la certification biologique, tout en ne présentant pas les inconvénients précités.

Selon l'invention, ces objets sont atteints au moyen d'un procédé d'obtention d'un extrait végétal consistant à :
i) mettre en contact au moins un matériau végétal avec un solvant ou un mélange de solvants comprenant au moins un alcane linéaire volatil d'origine végétale; et
ii) éliminer les résidus de matériau végétal, notamment par filtration.

Outre les avantages discutés dans le corps de la description détaillée qui suit, le procédé selon l'invention à l'avantage d'être simple et économique à mettre en oeuvre.

### Solvant d'extraction

Selon l'invention, le procédé d'extraction est mis en oeuvre au moyen d'au moins un solvant constitué d'au moins un alcane linéaire volatile d'origine végétale.

Selon un mode de réalisation, un alcane linéaire volatil convenant à l'invention peut présenter un point éclair compris dans l'intervalle variant de 30 à 120 °C, et plus particulièrement de 40 à 100 °C,.

Un alcane linéaire volatil convenant à l'invention est liquide à température ambiante (environ 25 °C) et à la pression atmosphérique (760 mmHg).

Selon un mode de réalisation, un alcane convenant à l'invention peut être un alcane linéaire volatil comprenant de 9 à 17 atomes de carbone, en particulier de 10 à 15 atomes de carbone, et plus particulièrement de 11 à 14 atomes de carbone.

Typiquement, l'alcane linéaire volatil ou le mélange d'alcanes linéaires volatils présent dans la composition selon l'invention comprend au moins un isotope 14C du carbone (carbone 14), en particulier l'isotope 14C peut être présent en un ratio 14C / 12C supérieur ou égal à 1.10-16, de préférence supérieur ou égal à 1.10-15, de préférence encore supérieur ou égal 7,5.10-14, et mieux supérieur ou égal 1,5.10-13. De préférence, le ratio 14C / 12C va de 6.10-13 à 1,2.10-12.

La quantité de d'isotopes 14C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcane convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcane linéaire d'origine végétale convenant à l'invention, on peut citer le n-nonane (C9), le n-décane (C10), le n-undécane (C11), le n-dodécane (C12), le n-tridécane (C13), le n-tétradecane (C14), le n-pentadécane (C15), le n-hexadecane (C16), le n-heptadecane (C17) et leurs mélanges, et en particulier le mélange de n-undécane (C11) et de n-tridécane (C13) obtenu dans les exemples 1 ou 2 de la demande WO2008/155059 de la Société Cognis, le n-dodécane (C12) et le n-tétradécane (C14) vendu par Sasol sous les références PARAFOL 12-97 et PARAFOL 14-97, respectivement dodécane et tétradécane linéaires ainsi que leurs mélanges.

On pourra utiliser l'alcane linéaire volatil seul ou en mélange d'au moins deux alcanes volatils distincts et différant entre eux d'un nombre de carbone d'au moins 1.

Selon un premier mode de réalisation, on utilise un mélange d'au moins deux alcanes linéaires volatils distincts comportant de 10 à 15 atomes de carbone et différant entre eux d'un nombre de carbone d'au moins 1. A titre d'exemples, on peut citer notamment les mélanges d'alcanes linéaires volatils C10/C11, C11/C12, C12/C13, ou C14/C15.

Selon un autre mode de réalisation, on utilise un mélange d'au moins deux alcanes linéaires volatils distincts comportant de 10 à 15 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et les mélanges C11/C13, ou C13/C15 pour un nombre de carbone n impair.

Selon un mode préféré, on utilise un mélange d'au moins deux alcanes linéaires volatils comportant de 10 à 15 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que par exemple un mélange d'au moins 3 alcanes linéaires volatils comportant de 9 à 15 atomes de carbone distincts et différant entre eux d'un nombre de carbone d'au moins 1, font également partie de l'invention, mais les mélanges de 2 alcanes linéaires volatils selon l'invention sont préférés (mélanges binaires).de préférence plus de 95% et mieux plus de 99% en poids de la teneur totale en alcanes linéaires volatils dans le mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cn avec n allant de 9 à 15
- de 10 à 50% en poids, de préférence de 20 à 45% en poids, de préférence de 24 à 40% en poids, d'alcane linéaire volatil en Cn+x avec x supérieur ou égal à 1n de préférence x=1 ou x=2, par rapport au poids total des alcanes dans ledit mélange.

En particulier, ledit mélange d'alcanes selon l'invention contient :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0.1% en poids d'hydrocarbures insaturés dans le mélange.

Plus particulièrement, un alcane linéaire volatil convenant à l'invention peut être mis en oeuvre sous la forme d'un mélange n-undécane/n-tridécane.

En particulier, on utilisera un mélange d'alcanes linéaires volatils comprenant :
- de 55 à 80% en poids, de préférence de 60 à 75% en poids d'alcane linéaire volatil en C11 (n-undécane)
- de 20 à 45% en poids, de préférence de 24 à 40% en poids d'alcane linéaire volatil en C13 (n-tridécane)
par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane

Selon un mode de réalisation particulier, un solvant hydrocarboné volatil convenant à l'invention peut présenter une vitesse d'évaporation inférieure ou égale à 0,13 mg/cm2/min.

Selon un mode de réalisation, un solvant hydrocarboné volatil convenant à l'invention peut présenter une vitesse d'évaporation comprise dans l'intervalle variant de 0,05 à 0,13 mg/cm2/min, en particulier de 0,08 à 0,12 mg/cm2/min, et plus particulièrement de 0,1 à 0,12 mg/cm2/min.

La volatilité d'un solvant hydrocarboné volatil conforme à l'invention peut être notamment évaluée au moyen du protocole décrit dans WO 06/013413, et plus particulièrement au moyen du protocole décrit ci-après.

On introduit dans un cristallisoir (diamètre : 7 cm) placé sur une balance se trouvant dans une enceinte d'environ 0,3 m3 régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

Les vitesses d'évaporation sont exprimées en mg de solvant hydrocarboné volatil évaporé par unité de surface (cm²) et par unité de temps (minute).

Le solvant hydrocarboné volatil selon l'invention a une vitesse d'évaporation inférieure ou égale à 0,13 mg/cm2/min. Cela correspond donc à une quantité de solvant évaporé inférieure ou égale à 3,9 mg/ cm² en 30 minutes.

Bien que ces solvants d'origine végétale présentent toutes les caractéristiques requises en termes d'innocuité, il peut être nécessaire d'éliminer toute trace de ceux-ci des extraits végétaux obtenus pour obtenir les actifs sous forme pure.

Les alcanes utilisés selon l'invention peuvent être éliminés par différentes techniques : par entraînement azéotropique à la vapeur d'eau sous vide ou par une distillation sous vide.

Ces solvants peuvent ensuite être facilement recyclés en fin de procédé pour d'autres extractions.

L'étape de mise en contact du matériau végétal avec le solvant peut se faire pendant une durée allant de 2 à 48h et de préférence, de 2 à 24h. Cette étape peut être raccourcie en procédant à l'extraction en présence d'un rayonnement ultrasons ou micro-ondes.

La mise en contact du matériau végétal avec le solvant ou le mélange de solvants peut se faire à une température d'extraction allant de 20 à 65°C.

La mise en contact du matériau végétal avec le solvant ou le mélange de solvant peut se faire par macération. D'autres techniques encore peuvent être utilisées. On citera notamment le trempage ou la pulvérisation.

A titre indicatif, le ratio en poids entre le matériau végétal et le solvant ou mélange de solvants va de 1 : 4 à 1 :10.

Le procédé selon l'invention peut comprendre en outre une étape visant à l'élimination totale ou partielle du ou des solvants résiduels. Une telle élimination peut se faire par entraînement azéotropique à la vapeur d'eau sous vide ou par une distillation sous vide.

Le matériau végétal utilisé dans le procédé selon l'invention peut être issu de feuilles, de tiges, de racines, de radicelles, de rhizomes, de bougeons, de fruits, de fleurs, de capitules de fleurs, de grains, de graines, de noyaux, de pépins, d'écorces, de mousses, de fragments de troncs, d'au moins une espèce végétale.

A titre purement illustratif, sont citées ci-après quelques exemples de plantes pouvant être utilisées dans le procédé selon l'invention : Branches de romarin frais (Rosmarinus Offinalis) ; Pétales de rose de Damas fraîche (Rosa Damascena) ; Fleurs fraîche de jasmin (Jasminum Officinalis); Feuilles de thé fraiche ou sèche (Camellia Sinensis) ; Graines de bourache (Borago Officinalis); Poudre de racines de curcuma (Curcuma Longa)

Selon un autre aspect, l'invention vise aussi un extrait végétal obtenu par le procédé selon l'invention, ledit extrait contenant une quantité mesurable d'au moins un alcane linéaire volatile. Le dosage d'alcane(s) linéaire(s) volatile(s) dans l'extrait selon l'invention peut être fait par toute méthode adéquate. On utilisera de préférence la chromatographie en phase gazeuse utilisant un détecteur à ionisation de flamme.

### Composition cosmétique ou dermatologique :

Selon encore un autre aspect, l'invention vise une composition cosmétique ou dermatologique contenant dans un milieu physiologiquement acceptable un extrait obtenu par le procédé d'extraction selon l'invention.

Selon un mode de réalisation, une composition de l'invention est substantiellement constituée de composés naturels ou d'origine naturelle.

De préférence, une composition selon l'invention peut comprendre de 1 % à 50 % en poids d'un solvant ou mélange de solvants comprenant au moins un alcane linéaire volatil, en particulier de 5 à 35 % en poids d'alcane(s) linéaire(s) volatil(s), et plus particulièrement de 8 à 25 % en poids d'alcane(s) linéaire(s) volatil(s) par rapport au poids total de la composition.

Pour la description de ce solvant ou de ce mélange de solvants on se réfèrera à la description faite plus haut en référence au solvant d'extraction.

Avantageusement, on privilégiera le même solvant ou mélange de solvants que celui utilisé pour l'extraction.

Une composition de l'invention peut comprendre en outre au moins un actif cosmétique et/ou un actif dermatologique.

A titre d'exemples, non limitatifs, d'actifs cosmétiques et/ou dermatologiques convenant à l'invention ou peut citer les actifs choisis parmi les agents suivants : les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents tenseurs, les agents liporestructurants, les agents amincissants, les agents favorisant la microcirculation cutanée, les agents apaisants et/ou anti-irritants, les sébo-régulateurs ou anti-séborrhéiques, les agents astringents, les agents cicatrisants, les agents anti-inflammatoires, les agents anti-acné, les agents matifiants, les charges à effet flouteur, les agents fluorescents, les agents favorisant la coloration naturellement rosée de la peau, les charges abrasives ou exfoliantes, les agents anti-microbiens, et les agents apaisants, les agents anti-NO, les agents anti-radicalaires ou anti-pollution, les filtres solaires, les vitamines telles que le tocophérol, les agents odorants telles que les huiles essentielles, les parfums, et leurs mélanges.

Les actifs cosmétiques et/ou dermatologiques convenant à l'invention peuvent notamment être choisis parmi les agents mentionnés dans la demande EP 1 847 247.

Une composition cosmétique selon l'invention peut également comprendre en outre tout additif usuellement utilisé dans le domaine concerné, par exemple choisi parmi des agents filmogènes, et le cas échéant, des auxiliaires de filmification, des gommes, des polymères semi-cristallins, des agent antioxydants, des huiles essentielles, des conservateurs, des parfums, des neutralisants, des agents antiseptiques, des agents protecteurs contre les UV, et leurs mélanges.

Il relève des opérations de routine de l'homme de l'art d'ajuster la nature et la quantité des additifs présents dans les compositions conformes à l'invention, de telle sorte que les propriétés cosmétiques, de confort à l'application et de stabilité désirées de celles-ci n'en soient pas affectées.

Une composition de l'invention peut être obtenue par tout procédé de préparation connu de l'homme de l'art.

### Exemple 1 :

On pèse 31.7g de feuille de violette (Viola Odorata ) fraîche préalablement hachées dans un récipient et on y ajoute 60g d'un mélange n-undécane/n-tridécane dans un rapport pondéral de 76 :24 tel que préparé selon l'exemple 1 de la demande WO2008/115059. L'ensemble est chauffé à 45°C durant 12h sous agitation régulière. L'ensemble est laissé refroidir à température ambiante puis filtré.

On obtient une solution de couleur vert foncé qui peut être utilisé comme extrait végétal dans une préparation cosmétique par exemple une huile pour le corps.

### Exemple 2 :

On pèse 35.1g de poudre de racine de chicorée torréfiée (Cichorium Intybus) dans un récipient et on y ajoute 70g d'un mélange n-undécane/n-tridécane dans un rapport pondéral de 76 :24 tel que préparé selon l'exemple 1 de la demande WO2008/115059. L'ensemble est chauffé à 55°C durant 10h sous agitation régulière. L'ensemble est laissé refroidir à température ambiante puis filtré.

On obtient une solution de couleur jaune foncé dont l'odeur est caractéristique qui peut être utilisé comme extrait végétal dans une préparation cosmétique par exemple une crème riche pour le corps.

### Exemple 3 :

On pèse 25.1 g de poudre de graines de café torréfiée (Coffea Arabica) dans un récipient et on y ajoute 65g d'un alcane linéaire volatil en C12 fourni par la société Sasol. L'ensemble est chauffé à 55°C durant 13h sous agitation régulière. L'ensemble est laissé refroidir à température ambiante puis filtré.

On obtient une solution de couleur brun clair dont l'odeur est caractéristique du café qui peut être utilisé comme extrait végétal dans une préparation cosmétique par exemple une pommade pour coudes et genoux secs.

L'extrait selon l'invention peut être intégré dans une composition cosmétique plus complexe :

### Exemple 4 : Soin Amincissant à l'extrait de café (extrait obtenu selon l'invention) :

| Eau | qsp100% |
|---|---|
| Acrylates/C10-30 alkyl acrylate crosspolymer (Pemulen TR1) | 0.6% |
| Triéthanolamine | 0.6% |
| Caféine | 2% |
| Ethanol | 20% |
| Glycérine | 3% |
| Butylène glycol | 2% |
| Octyldodecanol | 5% |
| Mélange de Undecane/Tridecane (Cetiol UT) | 4% |
| Extrait de café de l'exemple 3 | 5% |

## Revendications

1. Procédé d'obtention d'un extrait végétal consistant à :
i) mettre en contact au moins un matériau végétal avec un solvant ou un mélange de solvants comprenant au moins un alcane linéaire volatil d'origine végétale; et
ii) éliminer les résidus de matériau végétal, notamment par filtration.

2. Procédé selon la revendication 1 **caractérisé en ce que** ledit au moins un alcane linéaire volatil d'origine végétale est choisi parmi le n-nonane, le n-decane, le n-undécane, le n-dodécane, le n-tridécane, le n-tétradecane, le n-pentadecane, le n-hexadecane, le n-heptadecane, et leurs mélanges.

3. Procédé selon la revendication 1 ou 2 **caractérisé en ce que** ledit au moins un alcane linéaire volatil d'origine végétale comprend un mélange comprenant :
- de 55 à 80% en poids, de préférence de 60 à 75% en poids, préférentiellement encore de 65 à 70% en poids d'alcane linéaire volatil en C11 (n-undécane)
- de 20 à 45% en poids, de préférence de 24 à 40% en poids, préférentiellement encore de 24 à 30% en poids d'alcane linéaire volatil en C13 (n-tridécane)
par rapport au poids total des alcanes dans ledit mélange.

4. Procédé selon l'une quelconque des revendications 1 à 3 **caractérisé en ce que** la mise en contact se fait pendant une durée allant de 2 à 48h et de préférence, de 2 à 24h.

5. Procédé selon l'une quelconque des revendications 1 à 4 **caractérisé en ce que** la mise en contact du matériau végétal avec le solvant ou le mélange de solvants d'origine végétale se fait à une température d'extraction allant de 20 à 65°C.

6. Procédé selon l'une quelconque des revendications 1 à 5 **caractérisé en ce que** pendant l'étape de mise en contact, on soumet l'ensemble à un rayonnement micro-ondes ou ultrasons de manière à réduire le temps de mise en contact.

7. Procédé selon l'une quelconque des revendications 1 à 6 **caractérisé en ce que** la mise en contact du matériau végétal avec le solvant ou le mélange de solvant d'origine végétale se fait par macération.

8. Procédé selon l'une quelconque des revendications 1 à 7 **caractérisé en ce que** la mise en contact se fait par trempage ou pulvérisation.

9. Procédé selon l'une quelconque des revendications précédentes **caractérisé en ce que** le ratio en poids entre le matériau végétal et le solvant ou mélange de solvants d'origine végétale va de 1 : 4 à 1 :10.

10. Procédé selon l'une quelconque des revendications 1 à 9 **caractérisé en ce qu'**il comprend en outre une étape visant à l'élimination totale ou partielle du ou des solvants résiduels.

11. Procédé selon la revendication 10 **caractérisé en ce que** l'élimination totale ou partielle du ou des solvants résiduels se fait par entraînement azéotropique à la vapeur d'eau sous vide ou par une distillation sous vide.

12. Procédé selon l'une quelconque des revendications 1 à 11 **caractérisé en ce que** le matériau végétal est issu de feuilles, de tiges, de racines, de radicelles, de rhizomes, de bougeons, de fruits, de fleurs, de capitules de fleurs, de grains, de graines, de noyaux, de pépins, d'écorces, de mousses, de fragments de troncs, d'au moins une espèce végétale.

13. Extrait végétal obtenu par le procédé selon l'une quelconque des revendications 1 à 12, ledit extrait contenant une quantité mesurable d'au moins un alcane linéaire volatile d'origine végétale.

14. Composition cosmétique ou dermatologique contenant dans un milieu physiologiquement acceptable un extrait selon la revendication 13.

15. Composition cosmétique ou dermatologique selon la revendication 14 **caractérisé en ce qu'**elle comprend de 1 % à 50 % en poids d'un solvant ou mélange de solvants comprenant au moins un alcane linéaire volatil d'origine végétale, en particulier de 5 à 35 % en poids d'alcane(s) linéaire(s) volatil(s), et plus particulièrement de 8 à 25 % en poids d'alcane(s) linéaire(s) volatil(s) par rapport au poids total de la composition..
